# EUROPEAN PATENT SPECIFICATION

(11) **EP 3 892 719 B1**
(45) Date of publication and mention of the grant of the patent: **05.02.2025**
(21) Application number: 19891839.3
(22) Date of filing: 06.12.2019
(51) Int. Cl.: C12N 5/0735, C12N 5/10, C12N 5/00

(54) **UNDIFFERENTIATED STATE-MAINTAINING CULTURE MEDIUM FOR PLURIPOTENT STEM CELLS**
UNDIFFERENZIERTES ZUSTANDSERHALTENDES KULTURMEDIUM FÜR PLURIPOTENTE STAMMZELLEN
MILIEU DE CULTURE MAINTENANT L'ÉTAT DE NON DIFFÉRENTIATION DESTINÉ À DES CELLULES SOUCHES PLURIPOTENTES

(30) Priority: 07.12.2018 JP 2018230014
(43) Date of publication of application: 13.10.2021
(73) Proprietor: Kanto Kagaku Kabushiki Kaisha, Tokyo 103-0023 (JP)
(72) Inventor: WATANABE, Takehiro, Isehara-shi, Kanagawa 259-1146 (JP); SAWAGUCHI, Tomoya, Isehara-shi, Kanagawa 259-1146 (JP); YAMAZAKI, Megumi, Isehara-shi, Kanagawa 259-1146 (JP)
(74) Representative: Meissner Bolte Partnerschaft mbB
(86) International application number: PCT/JP2019/047857
(87) International publication number: WO 2020/116623

(56) References cited:
- WO-A1-2015/147047
- WO-A1-2015/147047
- WO-A1-2017/156762
- CN-A- 106 520 690
- CN-A- 107 267 462
- SAMPAZIOTIS FOTIOS ET AL: "Directed differentiation of human induced pluripotent stem cells into functional cholangiocyte-like cells", NATURE PROTOCOLS, NATURE PUBLISHING GROUP, GB, vol. 12, no. 4, 23 March 2017 (2017-03-23), pages 814 - 827, XP037551051, ISSN: 1754-2189, [retrieved on 20170323], DOI: 10.1038/NPROT.2017.011
- LUDOVIC VALLIER ED - BOEGEL ET AL: "Serum-Free and Feeder-Free Culture Conditions for Human Embryonic Stem Cells", 30 November 2010, BIOINFORMATICS FOR CANCER IMMUNOTHERAPY : METHODS AND PROTOCOLS; EMBRYONIC STEM CELL THERAPY FOR OSTEO-DEGENERATIVE DISEASES : METHODS AND PROTOCOLS; [METHODS IN MOLECULAR BIOLOGY, METHODS AND PROTOCOLS; ISSN 1064-3745], SPRINGER, NEW YORK, NY, PAGE(, ISBN: 978-1-0716-0326-0, XP009528774
- "Methods in molecular biology", vol. 690, 2011, SPRINGER SCIENCE+BUSINESS MEDIA, LLC, US, ISBN: 978-1-60761-961-1, article LUDOVIC VALLIER: "Serum-Free and Feeder-Free Culture Conditions for Human Embryonic Stem Cells", pages: 57 - 66, XP009528774, DOI: 10.1007/978-1-60761-962-8_3
- SAMPAZIOTIS, F. ET AL.: "Directed differentiation of human induced pluripotent stem cells into functional cholangiocyte-like cells", NATURE PROTOCOLS, vol. 12, no. 4, 23 March 2017 (2017-03-23), pages 814 - 827, XP055534582, DOI: 10.1038/nprot.2017.011

## Description

### [Technical Field]

The present invention relates to a medium for maintaining undifferentiated state for pluripotent stem cells.

### [Background Arts]

Pluripotent stem cells such as induced pluripotent stem cells (iPS cells) and embryonic stem cells (ES cells) possess pluripotency in differentiation and replication competence, so that they have been expected to have various utilization in fields of regenerative medicine, medical research, etc., especially in regenerative medicine. Culturing of a pluripotent stem cell requires proliferating it under a safe condition while maintaining its undifferentiated state.

For conventional cell culture, cell culture media comprising serum or albumin have been used. Albumin could be either of biological origin or recombinant. There is a concern that a medium comprising albumin of biological origin might contain a virus, etc. if the doner had been infected with such virus, etc. Even when the doner is not infected with such virus, etc., it could happen that certain factor being mixed during purification of the albumin is carried in the medium, and such factor might cause the cell to differentiate at an unintended timing. In addition, because it is of biological origin, there may be lot-to-lot differences in concentrations of minor components and quality, and such differences can change the culture results. On the other hand, recombinant albumin is expensive in cost. Therefore, in culturing of a pluripotent stem cell, it is preferred to use a medium which does not comprise albumin.

In recent years, development of a serum-free medium which is suitable for culturing a pluripotent stem cell has been in progress. Patent Reference 1 discloses a serum-free medium that comprises a ligand for endothelial differentiation gene (Edg) family receptor and a ligand for serotonin receptor. Patent Reference 2 discloses a serum-free medium that comprises a Pluronic non-ionic surfactant and animal hydrolysates but that does not comprise albumin. Patent Reference 3 discloses a method for culturing an embryonic stem cell while preventing its adherence to the culture dish by using a medium that comprises polyvinyl alcohol but which does not comprise albumin. Patent Reference 4 discloses that a synthetic polymer such as polyvinyl alcohol suppresses the adherence of a cell including an embryonic stem cell to the surface of a cell-culture substrate. Patent Reference 5 discloses a medium that comprises albumin and polyvinyl alcohol. Patent document 6 describes a serum-free medium and a method for the rapid production of induced pluripotent stemm cells, comprising a basal medium and an additive, wherein the medium comprises Porcine HGF, transferrins, FTN, polyvinylpyrrolidone, Genistein, mannitol, insulin, Glu, palmitic acid and hesperidine in specified amounts.
Non-Patent document 1 discloses a media composition and methods for culturing human induced pluripotent cells. The document specifically teaches that hPSCs can be cultured in CDM-PVA media supplemented with activin (10 ng/ml) and FGF (12 ng/ml). Non-Patent document 2 teaches a protocol for culturing human embryonic cells and human induced pluripotent stem cells. The culture media in this document includes i.a. Insulin, transferrin, bFGF and BSA. In addition, it is stated that BSA can be substituted with PVA.

However, there has been no medium that has a sufficient quality for a pluripotent stem cell, having a high proliferating property, being suitable for adhesive culture, etc., and having a wide applicability, without including albumin in the medium.

### [Prior Art References]

### [Patent References]

[Patent Reference 1] WO 2009/084662
[Patent Reference 2] WO 2017/195745
[Patent Reference 3] JP A 2007-228815
[Patent Reference 4] JP A 2007-124982
[Patent Reference 5] JP B 6197947
[Patent Reference 6] CN 107 267 462 A[Non-Patent Reference 1] Sampaziotis et al, NATURE PROTOCOLS, NATURE PUBLISHING GROUP, GB, vol. 12, no. 4, 2017, pp. 814-827
[Non-Patent Reference 1] Ed. LUDOVIC VALLIER, BIOINFORMATICS FOR CANCER IMMUNOTHERAPY 2010; ISSN 1064-3745814-827

### [Disclosure of Invention]

### [Problems to be Solved by the Invention]

Accordingly, the present invention is aimed at providing a medium for adhesive-culturing of a pluripotent stem cell that exhibits a high proliferating property without containing serum or albumin in the medium, and a widely-applicable method for adhesive-culturing of a pluripotent stem cell using such medium.

### [Means to Solve the Problems]

The present inventors have searched for non-albumin ingredients that provides a pluripotent stem cell with adherence to the surface of a cell-culture substrate and found that a hydrophilic polymer increases the adherence of the pluripotent stem cell to the surface of the cell-culture substrate and that an addition of such polymer into a medium would provide a high proliferating property in adhesive culturing of the pluripotent stem cell without including albumin in the medium. The present inventors further proceeded the investigation and reached the completion of the invention.

Namely, the present invention relates to a medium for adhesive culture of a pluripotent stem cell as specified in claim 1.

### [Effects of the Invention]

According to the present invention, by including a hydrophilic polymer in the medium, a pluripotent stem cell can be cultured at a high proliferating rate while maintaining its undifferentiated state without including albumin in the medium. Furthermore, by adding N-acetyl-L-cystein as an antioxidant into such medium, a higher proliferating rate can be achieved, so that a pluripotent stem cell can efficiently be cultured which is used in regenerative medicine or medical research field. Moreover, according to the method of the present invention, a method for enhancing the adherence of a cell can be provided in a medium that does not comprise albumin.

### [Brief Description of Drawings]

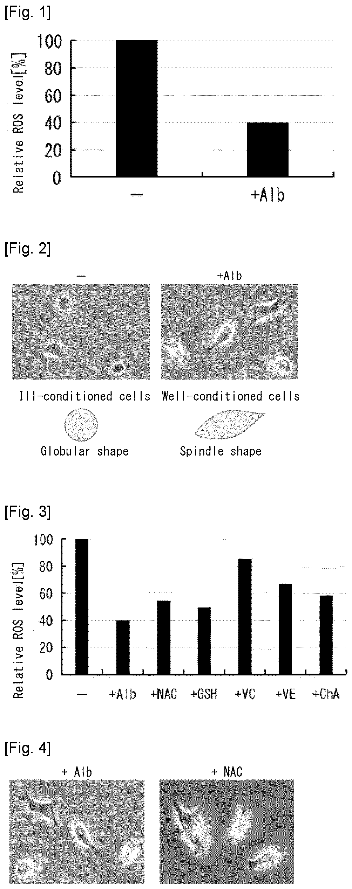

[Fig. 1] Fig. 1 shows the relevance of intracellular reactive oxygen species (ROS) level in iPS cells cultured either in albumin-added medium or in a medium with no additive in Comparative Example 1. "-" indicates the result of the culturing in the medium with no added albumin; "+Alb" indicates the result of the culturing in the albumin-added medium. The value of each result in the drawing indicates the relative value when the intracellular ROS level in the medium with no additive is considered as 100 %.
[Fig. 2] Fig. 2 shows phase-contrast images of iPS cells on the third day of culturing either in the albumin-added medium or in the medium with no additive in Comparative Example 1. In the drawing, "-" indicates the image of the cells cultured in the medium to which no albumin had been added; "+Alb" indicates the image of cells cultured with added albumin. The cells take spindle morphology in good condition, whereas they take globular morphology when the condition goes wrong.
[Fig. 3] Fig. 3 shows the relationship between the antioxidant and intracellular ROS level when iPS cells were cultured either in the medium into which an antioxidant had been added or in the medium with no additive in Example 1. In the drawing, "+Alb" indicates the result of the culturing cells in a medium to which albumin had been added, "+NAC" for a medium to which N-acetyl-L-cysteine had been added, "+GSH" for a medium to which reduced glutathione had been added, "+VC" for a medium to which vitamin C had been added, "+VE" for a medium to which vitamin E had been added, and "+ChA" for a medium to which chlorogenic acid had been added. The value of each result in the drawing indicates the relative value to when the intracellular ROS level in the medium with no additive is considered as 100 %.
[Fig. 4] Fig. 4 shows phase-contrast images of iPS cells cultured for 3 days either in the albumin-added medium or in the N-acetyl-L-cysteine-added medium in Example 2. "+Alb" indicates the image of the cells cultured in the medium to which albumin had been added; "+NAC" indicates the image of the cells cultured in the medium to which N-acetyl-L-cysteine had been added.

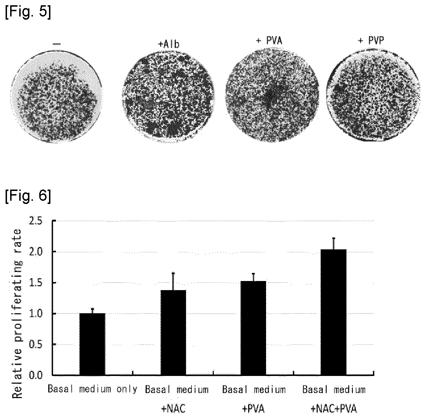

[Fig. 5] Fig. 5 shows phase-contrast microscopy images of the whole culture containers when all cells were stained blue with crystal violet in Example 3. "-" indicates the result of culturing in the medium with no additive. "+Alb" indicates the result of culturing in the albumin-added medium. "+PVA" indicates the result of culturing in the medium with polyvinyl alcohol being added. "+PVP" indicates the result of culturing in the medium with polyvinylpyrrolidone being added.
[Fig. 6] Fig. 6 shows the relative proliferating rate (mean value ± standard deviation) in Example 4 when being cultured for 7 days in the basal medium alone, in the basal medium to which only N-acetyl-L-cysteine had been added, in the basal medium to which only polyvinyl alcohol had been added, or in the basal medium to which N-acetyl-L-cysteine and polyvinyl alcohol had been added. In the drawing, "basal medium+NAC" indicates the basal medium to which only N-acetyl-L-cysteine had been added, "basal medium+PVA" indicates the basal medium to which only polyvinyl alcohol had been added, and "basal medium+NAC+PVA" indicates the basal medium to which N-acetyl-L-cysteine and polyvinyl alcohol had been added. The numerical values on the vertical axis in the drawing refer to relative proliferating rates when that of case using only the basal medium was considered as 1.

### [Mode for Carrying out the Invention]

Hereinbelow, the present invention will be explained in detail based on suitable embodiments of the present invention.

The "medium for adhesive culture of a pluripotent stem cell" according to the present invention is a medium that is used for culturing a pluripotent stem cell in an adhesive manner. The "pluripotent stem cell" according to the present invention is a cell that has the pluripotency in differentiation and replication competence such that the cell is capable of differentiating into any tissue or cell that constitutes an organism. Examples include ES cells, embryonic germ cells (EG cells) and iPS cells, and iPS cells are preferred. Biological species from which the "pluripotent stem cell" according to the present invention is derived are not particularly limited, and include, for example, human, monkeys and mouse. The cell is preferably of human-origin.

The medium used in the present invention comprises a hydrophilic polymer. The present inventors have found that, in a medium that comprise substantially no albumin, the hydrophilic polymer enhances the adherence of a cell to a culture dish and promotes cell proliferation in adhesive-culturing. Although not being bound by a theory, it is assumed that the hydrophilic polymer that is added into the medium of the present invention forms a film on the surface of the cell adhered to the culture substrate, thereby increasing the adherence of the cell. Therefore, the hydrophilic polymer that is used in the present invention is not limited as long as it increases the adherence of the cell to the culture substrate. Polyvinyl alcohol, polyvinylpyrrolidone, polyethylene glycol, polypropylene glycol, polyethylene imine, carboxymethyl cellulose, methyl cellulose, dextran, gellan gum, alginic acid, etc. may be used. In view of conferring a stronger adherence to the culture substrate, polyvinyl alcohol is preferred. Moreover, the hydrophilic polymer in the present invention preferably is a non-natural product and a highly safe hydrophilic polymer that has been employed in medical products, because it is to be used in regenerative medicine and in research in medical field.

The concentration of the hydrophilic polymer that is added into the medium of the present invention is not limited as long as the hydrophilic polymer is capable of increasing the adherence of a cell to a culture substrate at that concentration, for example between 0.01 % and 5 %, preferably between 0.05 % and 1 %. In particular, polyvinyl alcohol of 0.1 to 0.5 % and polyvinylpyrrolidone of 0.1 to 0.5 % are further preferred in view of being capable of enhancing the adherence.

The method of enhancing the adherence of a cell to a culture substrate according to the present invention comprises adding a hydrophilic polymer into a medium, wherein the hydrophilic polymer may be added at any timing during producing or using the medium. A known culture substrate may be used according to the present invention. For example, without limitation, a culture substrate of polystyrene resin or glass may be used, with or without a protein coating such as laminin, vitronectin, collagen, gelatin or casein, or with immobilized polymer.

Since a biologically-originated albumin might have lot-to-lot differences or contamination of a factor, the inventive medium equal to or less than 0.010 % of albumin. It is preferred that the inventive medium does not to include albumin at all, or even if albumin is included, it is preferred to be as little as possible. Therefore, in the medium of the present invention, there is either no albumin at all, or albumin in an extent in which it does not cause lot-to-lot difference or any influence by a factor, i.e., an extent in which it neither promote proliferation of a pluripotent stem cell nor exhibit a stability-maintaining effect. Such a medium comprises equal to or less than 0.010 % of albumin, preferably equal to or less than 10⁻⁶ % of albumin.

Known basal media may be used in the present invention, including, but not limited to, for example, DMEM (Dulbecco's modified Eagle medium), MEM (Eagle's minimum essential medium), α-MEM (Eagle's minimum essential medium α-modified type), GMEM (Glasgow minimum essential medium), Ham's F-12 (mixed nourishments F-12 Ham), DMEM/Ham, IMDM (Iscove's modified Dulbecco's medium) and DMEM/F-12 (Dulbecco's modified Eagle medium/mixed nourishments F-12 Ham). The medium preferably includes DMEM/F-12 and the like.

The components of the medium may include known additives as required. Additives may be those which do not inhibit cell proliferation, and various inorganic salts, carbohydrates, amino acids, vitamins and lipids which have conventionally been used for culturing of pluripotent stem cells may be included. Moreover, a protein component other than albumin may be included as an additive. The protein component other than albumin is, for example, a growth factor, an iron storage- or transport-factor, etc. Growth factors include, though not being limited to, epithelial growth factor(EGF), insulin-like growth factor (IGF), vascular endothelial cell growth factor (VEGF), thrombopoietin, transforming growth factor (TGF), basic fibroblast growth factor (bFGF), and insulin although it does not have a direct effect. The iron storage- or transport-factor include, though not being limited to, transferrin. More preferably, the proteins other than albumin are insulin, transferrin and bFGF. These proteins are added preferably such that the final concentration of insulin is between 1 and 50 µg/mL, the final concentration of transferrin is between 1 and 30 µg/mL, and the final concentration of bFGF is between 1 and 100 ng/mL, more preferably such that the final concentration of insulin is between 10 and 30 µg/mL, the final concentration of transferrin is between 5 and 20 µg/mL, and the final concentration of bFGF is between 2.5 and 25 ng/mL.

The medium of the present invention comprises N-acetyl-L-cysteine as an antioxidant. Without being bound by a theory, it is assumed that the antioxidant in the medium of the present invention would contribute to a reaction in which ROS, which is produced in large amount in a cell due to the stress added to the cell, is captured and thereby is detoxified. Therefore, the "antioxidant" that is used in the present invention contributes to a reaction in which ROS is captured and thereby is detoxified. According to the invention, the antioxidant is N-acetyl-L-cysteine,. The antioxidants is added preferably such that the final concentration of N-acetyl-L-cysteine is between 0.05 and 10 mM. Further preferably, the antioxidants are added such that the final concentration of N-acetyl-L-cysteine is between 0.3 and 1 mM.

In one embodiment of the present invention, the basal medium may comprise a differentiation suppressing agent as an additive. The differentiation suppressing agent includes, though not being limited to, GSK3β inhibitor and DYRK inhibitor, leukemia inhibitory factor (LIF), MEK inhibitor and GSK3 inhibitor. Preferably, the differentiation suppressing agents are GSK3β inhibitor and DYRK inhibitor. The GSK3β inhibitor has an effect of inhibiting the function of GSK3β or limiting its expression, and includes, though not being limited to, SB216763, BIO, AR-A014418, IM-12, CHIR99021, Kenpaullone, and 1-Azakenpaullone. The DYRK inhibitor has an effect of inhibiting the function of DYRK or limiting its expression, and includes, though not being limited to, AZ191, ID-8 and Harmine hydrochloride. Further preferably, the GSK3β inhibitor is 1-Azakenpaullone, and the DYRK inhibitor is ID-8. The differentiation suppressing agents are added preferably such that the final concentration of 1-Azakenpaullone is between 400 and 2000 nM and the final concentration of ID-8 is between 200 and 1000 nM. Further preferably, the antioxidants are added such that the final concentration of 1-Azakenpaullone is between 700 and 1400 nM and the final concentration of ID-8 is between 400 and 800 nM.

In one embodiment of the present invention, the basal medium may comprise an NFAT inhibitor. The NFAT inhibitor has an effect of inhibiting the function of NFAT or limiting its expression. The NFAT inhibitor includes, though not being limited to, Tacrolimus (FK506), Cyclosporin A, MCV-1 and INCA-6. Preferably, the NFAT inhibitor is Tacrolimus. The NFAT inhibitor is added preferably such that the final concentration of Tacrolimus is between 10 and 100 pM, further preferably such that the final concentration of Tacrolimus is between 10 and 50 pM.

The basal medium of the present invention may comprise a component other than those listed above as long as it does not inhibit the proliferation of the pluripotent stem cell. In view of efficiently expanding cells, it is selenium or ethanolamine, preferably selenium, and further preferably sodium selenite.

In one embodiment of the present invention, the medium is a medium for an adhesive culture, and it may be a basal medium comprising a hydrophilic polymer, an antioxidant, and a differentiation suppressing agent consisting of a GSK3β inhibitor and a DYRK inhibitor. The medium may further comprise known additives as needed. Additives may be those which do not inhibit cell proliferation, including various inorganic salts, carbohydrates, amino acids, vitamins, proteins and lipids which have conventionally been used for culturing pluripotent stem cells.

Culturing of a pluripotent stem cell using the medium of the present invention may be performed using any adhesive-culturing methods known to a skilled artisan. As an example, culturing may be performed as follows: pluripotent stem cells are washed with PBS, then cells were dissociated at 37 °C for 5 minutes to be dispersed into single-cells, collected and plated onto a laminin-coated 6-well plate for adhesive-culturing, cultured overnight at 37 °C under 5 % CO₂ condition in the medium of the present invention comprising a ROCK inhibitor, and cultured for 7 days while exchanging the medium as appropriate to one that does not comprise the ROCK inhibitor.

Hereinbelow, the present invention will be explained in further detail referring to Examples, though the present invention is not limited to these Examples.

### [Examples]

### [Comparative Example 1: Functional Validation of Albumin in Cell Culture]

### [Medium Preparation]

DMEM/F12 with trace element medium (from Sigma, D0547) 10.8 g, 1M HEPES (from ThermoScientific, 15630-080) 14 mL, sodium bicarbonate 1.1 g, sodium chloride 0.4 g, L-ascorbic acid phosphate magnesium salt (from FUJIFILM Wako Pure Chemical Corporation, 013-19641) 0.06 g, ITS solution (from ThermoScientific, 41400-045, Insulin 1 mg/mL, Transferrin 0.55 mg/mL, Selenium 0.7 pg/mL) 18 mL, 1-Azakenpaullone (from Toronto Research Chemicals, A800950) 361 µg, ID-8 (from Cayman Chemicals, 15222) 179 µg, and Tacrolimus (from Cayman Chemicals , 10007965) 16 ng were dissolved in 1 L of water, which were mixed to give 1 L of a basal medium.

To 100 mL of the basal medium, 1 g of albumin (from MP Biomedicals, 0215240180-100g) was added to give an albumin-added medium.

### [Measurement of ROS]

In each of the basal medium with no additive and the albumin-added medium, cells were dispersed into single-cells, and after 1 hours, for each case, cells were fluorescently stained with DCFH-DA, and fluorescence intensity was measured using a flow cytometer.

### [Results]

As shown in Fig. 1, it was confirmed that the intracellular ROS level was decreased by approximately 60 % by albumin addition.

### [Culture]

Human iPS cell line 253G1 was washed with PBS, then treated with an equivalent mixture solution of TrypLE Select (ThermoScientific) and 0.5 mM EDTA at 37 °C for 5 minutes to be dispersed into single-cells, and collected. The number of cells was counted using a hemocytometer, and then the cells were plated to be 0.25 µg/cm² onto a 6-well plate for adhesive-culturing coated with iMatrix-511 (Matrixome Inc.) at 25,000 cells/well, cultured overnight in the above-described medium comprising 10 µM Y-27632 (from Cayman Chemicals, 10005583) at 37 °C under 5 % CO₂ condition, and cultured for 7 days while exchanging the medium every day to one that does not comprise Y-27632.

The culture was performed in the basal medium with no additive as a control and in the medium which is the basal medium to which albumin had been added (n=1).

### [Confirmation of Cell Morphology]

The morphology of cells on the third day of culturing in the basal medium with no additive and in the albumin-added medium was observed by phase-contrast microscopy.

### [Results]

As shown in Fig. 2, the cell morphology in the albumin-added medium shows a spindle shape, confirming a better condition than the cell morphology in the medium with no additive which showed a globular shape.

### [Example 1: Validation of Influences of Antioxidant-Comprising Medium on Intracellular ROS Level]

### [Medium Preparation]

The basal medium was prepared in a similar way to in Comparative Example 1. To 100 mL of the basal medium, either N-acetyl-L-cysteine (from KANTO KAGAKU, 01763-30) 163 mg, reduced glutathione (from KANTO KAGAKU, 17501-61, not according to the invention) 307 mg, vitamin C (from FUJIFILM Wako Pure Chemical Corporation, 013-19641, not according to the invention) 278 mg, vitamin E (from KANTO KAGAKU, 40562-30, not according to the invention) 430 mg, or chlorogenic acid (from KANTO KAGAKU, 10924-1A, not according to the invention) 353 mg was added. As controls, the basal medium with no additive, and 100 mL of the basal medium to which 1 g of albumin was added were obtained.

### [Measurement of ROS]

Cells were cultured in the control media or in the medium to which the respective component had been added, then dispersed into single-cells. For each case, cells were fluorescently stained with DCFH-DA, and the fluorescence intensity was measured using a flow cytometer.

### [Results]

As shown in Fig. 3, a decrease in fluorescence intensity was confirmed in all of the cells cultured in the medium to which either of five types of antioxidants had been added, suggesting that the ROS level was decreased. Among the five types, a high effect was confirmed in N-acetyl-L-cysteine and reduced glutathione, confirming approximately 50 % decrease in intracellular ROS level.

### [Example 2: Culture of Pluripotent Stem Cell Using Antioxidant- Comprising Medium]

### [Medium Preparation]

The basal medium was prepared in a similar way to in Comparative Example 1. To 100 mL of the basal medium, either N-acetyl-L-cysteine (from KANTO KAGAKU, 01763-30) 8.2 mg, reduced glutathione (from KANTO KAGAKU, 17501-61, not according to the invention) 16 mg, vitamin C (from FUJIFILM Wako Pure Chemical Corporation, 013-19641, not according to the invention) 14 mg, vitamin E (from KANTO KAGAKU, 40562-30, not according to the invention) 21 mg or chlorogenic acid (from KANTO KAGAKU, 10924-1A, not according to the invention) 17 mg was added. As controls, the basal medium with no additive, and 100 mL of the basal medium with 1 g of added albumin were obtained.

### [Culture]

Human iPS cell line 253G1 was washed with PBS, then treated with an equivalent mixture solution of TrypLE Select (ThermoScientific) and 0.5 mM EDTA at 37 °C for 5 minutes to be dispersed into single-cells, and collected. The number of cells was counted using a hemocytometer, and then the cells were plated at 0.25 µg/cm² onto a 6-well plate for adhesive-culturing coated with iMatrix-511 (Matrixome Inc.) at 25,000 cells/well, cultured overnight in the above-described medium comprising 10 µM Y-27632 at 37 °C under 5 % CO₂ condition, and cultured for 7 days while exchanging the medium every day to one that does not comprise Y-27632.

The culture was carried out in the basal medium with no additive and an albumin-added medium which were prepared as controls, and in a medium to which N-acetyl-L-cysteine was added, in a medium to which reduced glutathione was added, in a medium to which vitamin C was added, in a medium to which vitamin E was added or in a medium to which chlorogenic acid was added (n=1).

### [Confirmation of Cell Morphology]

The morphology of cells on the third day of culturing in the control medium and in the N-acetyl-L-cysteine-added medium as a representative of 5 types of antioxidants was observed by phase-contrast microscopy.

### [Results]

As shown in Fig. 4, the morphology of the cells cultured in N-acetyl-L-cysteine-added medium also shows a spindle shape, confirming a good cell condition.

### [Example 3: Culture of Pluripotent Stem Cell Using Hydrophilic Polymer-Comprising Medium]

### [Medium Preparation]

The basal medium was prepared in a similar way to in Comparative Example 1. To 100 mL of the basal medium, either polyvinyl alcohol (from Sigma, P8136) 0.25 g, polyvinylpyrrolidone (from Sigma, PVP40) 0.25 g or 1 g of albumin was added.

### [Culture]

Human iPS cell line 253G1 was washed with PBS, then treated with an equivalent mixture solution of TrypLE Select (ThermoScientific) and 0.5 mM EDTA at 37 °C for 5 minutes to be dispersed into single-cells, and collected. The number of cells was counted using a hemocytometer, and then the cells were plated at 0.25 µg/cm² onto a 6-well plate for adhesive-culturing coated with iMatrix-511 (Matrixome Inc.) at 25,000 cells/well, cultured overnight in the above-described medium comprising 10 µM Y-27632 at 37 °C under 5 % CO₂ condition, and cultured for 7 days while exchanging the medium every day to one that does not comprise Y-27632.

The culture was carried out in the basal medium with no additive and an albumin-added medium prepared as controls, and in a medium to which polyvinyl alcohol and in a medium to which polyvinylpyrrolidone was added (n=3).

### [Confirming Cell Attachment]

Each of the cultured cell was stained with crystal violet and the adherence of the cells was confirmed under phase-contrast microscopy.

### [Results]

As shown in Fig. 5 (-), in the medium with no additive, the stained cells were confirmed only in the center of the culture container, whereas were confirmed no cell attaching on the periphery of the culture container. On the other hand, as shown in Fig. 5 (+Alb), in the albumin-added medium, stained cells were confirmed all over the culture container, confirming that there was no detachment of cell. Moreover, as shown in Fig. 5 (+PVA) and (+PVP), in cells cultured in the medium to which either of the two hydrophilic polymers was added, it was confirmed that more cells attached all over the culture container to its periphery as compared to the cells that were cultured in a condition where neither of the hydrophilic polymers was added.

### [Example 4: Comparison of Proliferating Rate in Media with Each Added Components]

### [Medium Preparation]

The basal medium was prepared in a similar way to in Comparative Example 1.

To 100 mL of the basal medium, either only 8.2 mg of N-acetyl-L-cysteine was added, only 0.25 g of polyvinyl alcohol was added, or 8.2 mg of N-acetyl-L-cysteine and 0.25 g of polyvinyl alcohol were added.

### [Culture]

Human iPS cell line 253G1 was washed with PBS, then treated with an equivalent mixture solution of TrypLE Select (ThermoScientific) and 0.5 mM EDTA at 37 °C for 5 minutes to be dispersed into single-cells, and collected. The number of cells was counted using a hemocytometer, and then the cells were plated at 0.25 µg/cm² onto a 6-well plate for adhesive-culturing coated with iMatrix-511 (Matrixome Inc.) at 25,000 cells/well, cultured overnight in the above-described medium comprising 10 µM Y-27632 at 37 °C under 5 % CO₂ condition, and cultured for 7 days while exchanging the medium every day to one that does not comprise Y-27632.

The culture was carried out in the basal medium with no additive prepared as a control, and in a medium to which only N-acetyl-L-cysteine was added, in a medium to which only polyvinyl alcohol was added, or in a medium to which N-acetyl-L-cysteine and polyvinyl alcohol were added (n=3).

### [Results]

As shown in Fig. 6, approximately 1.4-fold increase in relative proliferating rate was confirmed for the case where only N-acetyl-L-cysteine was added, approximately 1.5-fold increase for the case where only polyvinyl alcohol was added, approximately 2.0-fold increase for the case where both N-acetyl-L-cysteine and polyvinyl alcohol were added, as compared to the culture result using only the basal medium.

### [Industrial Applicability]

By using the medium of the present invention, it is now possible to efficiently and stably expand pluripotent stem cells without using albumin. The pluripotent stem cell cultured in such a medium can widely be used for regenerative medicine and research in medical field because it was not cultured using albumin. It is also less expensive and therefore can spread the regenerative medicine and drug development using pluripotent stem cells.

## Claims

1. A method for the adhesive culture of a pluripotent stem cell, comprising culturing the pluripotent stem cell in a medium comprising a hydrophilic polymer and equal to or less than 0.010 % of albumin, wherein the medium further comprises N-acetyl-L-cysteine as an antioxidant.

2. The method according to Claim 1, wherein the hydrophilic polymer in the medium is polyvinyl alcohol or polyvinylpyrrolidone.

3. The method according to Claim 1 or 2, wherein the medium further comprises a protein component other than albumin.

4. The method according to Claim 3, wherein the protein component other than albumin in the medium is at least one type selected from a group consisting of insulin, transferrin and basic fibroblast growth factor (bFGF).

5. The method according to any one of Claims 1 to 4, wherein the medium further comprises a differentiation suppressing agent.

6. The method according to Claim 5, wherein the differentiation suppressing agent in the medium is GSK3β (glycogen synthase kinase 3β) inhibitor, and/or DYRK (Dual-specificity tyrosine-phosphorylation-regulated kinase) inhibitor.

7. The method according to Claim 6, wherein the GSK3β inhibitor in the medium is 1-Azakenpaullone.

8. The method according to Claim 6, wherein the DYRK inhibitor in the medium is ID-8.

9. The method according to any one of Claims 1 to 8, wherein the medium further comprises NFAT (nuclear factor of activated T-cells) inhibitor.

10. The method according to Claim 9, wherein the NFAT inhibitor in the medium is Tacrolimus.

11. The method according to any one of Claims 1 to 10, wherein the pluripotent stem cell is derived from human.

12. The method according to any one of Claims 1 to 11, wherein the pluripotent stem cell is an induced pluripotent stem cell (iPS cell).

13. Use of a hydrophilic polymer in a medium for enhancing the adherence of a pluripotent stem cell to a culture substrate.

14. A medium for the adhesive culture of a pluripotent stem cell comprising a hydrophilic polymer, N-acetyl-L-cysteine, and equal to or less than 0.010 % of albumin.

## Patentansprüche

1. Verfahren für die Haftkultur einer pluripotenten Stammzelle, umfassend Kultivieren der pluripotenten Stammzelle in einem Medium, umfassend ein hydrophiles Polymer und gleich oder weniger als 0,010 % Albumin, wobei das Medium ferner N-Acetyl-L-Cystein als ein Antioxidans umfasst.

2. Verfahren nach Anspruch 1, wobei das hydrophile Polymer in dem Medium Polyvinylalkohol oder Polyvinylpyrrolidon ist.

3. Verfahren nach Anspruch 1 oder 2, wobei das Medium ferner eine andere Proteinkomponente als Albumin umfasst.

4. Verfahren nach Anspruch 3, wobei die andere Proteinkomponente als Albumin in dem Medium mindestens einer Art ist, die ausgewählt ist aus einer Gruppe bestehend aus Insulin, Transferrin und basischem Fibroblastenwachstumsfaktor (bFGF).

5. Verfahren nach einem der Ansprüche 1 bis 4, wobei das Medium ferner ein Spezialisierungsunterdrückungsmittel umfasst.

6. Verfahren nach Anspruch 5, wobei das Spezialisierungsunterdrückungsmittel in dem Medium ein GSK3β-Hemmer (Glykogensynthase-Kinase-3β-Hemmer) und/oder ein DYRK-Hemmer (dual spezifischer Tyrosin-Phosphorylierungsregulierter Kinase-Hemmer) ist.

7. Verfahren nach Anspruch 6, wobei der GSK3β-Hemmer in dem Medium 1-Azakenpaullon ist.

8. Verfahren nach Anspruch 6, wobei der DYRK-Hemmer in dem Medium ID-8 ist.

9. Verfahren nach einem der Ansprüche 1 bis 8, wobei das Medium ferner einen NFAT-Hemmer (Nuklearfaktor aktivierter T-Zellen-Hemmer) umfasst.

10. Verfahren nach Anspruch 9, wobei der NFAT-Hemmer in dem Medium Tacrolimus ist.

11. Verfahren nach einem der Ansprüche 1 bis 10, wobei die pluripotente Stammzelle von einem Menschen stammt.

12. Verfahren nach einem der Ansprüche 1 bis 11, wobei die pluripotente Stammzelle eine induzierte pluripotente Stammzelle (iPS-Zelle) ist.

13. Verwendung eines hydrophilen Polymers in einem Medium zum Verbessern der Haftung einer pluripotenten Stammzelle an einem Kultursubstrat.

14. Medium für die Haftkultur einer pluripotenten Stammzelle, umfassend ein hydrophiles Polymer, N-Acetyl-L-Cystein, und gleich oder weniger als 0,010 % Albumin.

## Revendications

1. Procédé de culture adhésive d'une cellule souche pluripotente, comprenant la mise en culture de la cellule souche pluripotente dans un milieu comprenant un polymère hydrophile et un pourcentage d'albumine égal ou inférieur à 0,010 %, dans lequel le milieu comprend en outre de la N-acétyl-L-cystéine en tant qu'antioxydant.

2. Procédé selon la revendication 1, dans lequel le polymère hydrophile dans le milieu est l'alcool polyvinylique ou la polyvinylpyrrolidone.

3. Procédé selon la revendication 1 ou 2, dans lequel le milieu comprend en outre un composant protéique autre que l'albumine.

4. Procédé selon la revendication 3, dans lequel le composant protéique autre que l'albumine dans le milieu est au moins un type choisi dans un groupe consistant en l'insuline, la transferrine et le facteur de croissance basique des fibroblastes (bFGF).

5. Procédé selon l'une quelconque des revendications 1 à 4, dans lequel le milieu comprend en outre un agent de suppression de différenciation.

6. Procédé selon la revendication 5, dans lequel l'agent de suppression de différenciation dans le milieu est un inhibiteur de GSK3β (glycogène synthase kinase 3β), et/ou un inhibiteur de DYRK (kinase à spécificité double régulée par tyrosine-phosphorylation).

7. Procédé selon la revendication 6, dans lequel l'inhibiteur de GSK3β dans le milieu est la 1-azakenpaullone.

8. Procédé selon la revendication 6, dans lequel l'inhibiteur de DYRK dans le milieu est l'ID-8.

9. Procédé selon l'une quelconque des revendications 1 à 8, dans lequel le milieu comprend en outre un inhibiteur de NFAT (facteur nucléaire des cellules T activées).

10. Procédé selon la revendication 9, dans lequel l'inhibiteur de NFAT dans le milieu est le tacrolimus.

11. Procédé selon l'une quelconque des revendications 1 à 10, dans lequel la cellule souche pluripotente est dérivée de l'homme.

12. Procédé selon l'une quelconque des revendications 1 à 11, dans lequel la cellule souche pluripotente est une cellule souche pluripotente induite (cellule iPS).

13. Utilisation d'un polymère hydrophile dans un milieu pour améliorer l'adhérence d'une cellule souche pluripotente à un substrat de culture.

14. Milieu pour la culture adhésive d'une cellule souche pluripotente comprenant un polymère hydrophile, de la N-acétyl-L-cystéine, et un pourcentage d'albumine égal ou inférieur à 0,010 %.
